(19) Europäisches Patentamt
European Patent Office
Office européen des brevets

(11) Veröffentlichungsnummer: **0 263 492 B1**

(12) # EUROPÄISCHE PATENTSCHRIFT

(45) Veröffentlichungstag der Patentschrift: **13.03.91**

(51) Int. Cl.$^5$: **C07C 403/00**, C07C 69/734, C07C 59/64, C07C 321/20, C07C 317/10, C07D 295/08, C07D 207/27, C07D 207/404, A61K 31/07

(21) Anmeldenummer: **87114587.6**

(22) Anmeldetag: **06.10.87**

(54) **Neue Retinoide, deren Herstellung und Verwendung als Pharmazeutika.**

(30) Priorität: **06.10.86 CH 3987/86**
**21.08.87 CH 3216/87**

(43) Veröffentlichungstag der Anmeldung:
**13.04.88 Patentblatt 88/15**

(45) Bekanntmachung des Hinweises auf die Patenterteilung:
**13.03.91 Patentblatt 91/11**

(84) Benannte Vertragsstaaten:
**AT BE CH DE FR GB IT LI LU NL SE**

(56) Entgegenhaltungen:
**WO-A-84/01573**
**GB-A- 1 543 825**
**US-A- 4 215 215**

(73) Patentinhaber: **F. HOFFMANN-LA ROCHE AG**
**Postfach 3255**
**CH-4002 Basel(CH)**

(72) Erfinder: **Gross, Günter, Dr.**
**Dinkelbergstrasse 23**
**W-7858 Weil a/Rhein(DE)**

(74) Vertreter: **Lederer, Franz, Dr. et al**
**Patentanwalt Dr. Franz Lederer Lucile-Grahn-Strasse 22**
**W-8000 München 80(DE)**

**EP 0 263 492 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue Retinoide der Formel

$$\text{Ret-OA} \qquad I$$

worin

- Ret der Acylrest einer Retinoidcarbonsäure, A ein Rest $(-CHR-CH_2O)_nR^1$, $(-CH_2)_mSR^1$ $(-CH_2)_mXR^2$, $(-CH_2)_m$-Het, $-N(R^2)_2$, $-C(R^4)_2OC(O)R^3$, $-CH_2-CH(OR^2)CH_2OR^2$ oder $-CH(CH_2OR^2)_2$;
- R Wasserstoff oder Methyl,
- $R^1$ Wasserstoff, $C_{1-6}$-Alkyl oder $C_{1-6}$ -Alkanoyl,
- $R^3$ $C_{1-6}$-Alkyl, $C_{1-6}$-Alkoxy, Di-$C_{1-6}$ -Alkylamino, $R^3$ $C_{1-6}$-Alkyl, $C_{1-6}$-Alkoxy, Di-$C_{1-6}$-Alkylamino, Phenyl, substituiertes Phenyl, Styryl oder im Phenylrest substituiertes Styryl oder 2-Benzoyl-phenoxy--$C_{1-6}$ -alkyl;
- $R^4$ Wasserstoff, $C_{1-6}$-Alkyl oder Phenyl;
- X >SO oder >$SO_2$,
- Het Pyrrolidino, Piperidino, Mono- oder Diketopyrrolidino, Mono- oder Diketopiperidino, Piperazino, $N^4$-$C_{1-4}$-Alkylpiperazino, Morpholino oder Thiamorpholino,
- n eine ganze Zahl von 3-40 und
- m eine ganze Zahl von 1-4 ist, wobei $R^1$ $C_{1-6}$-Alkyl oder $C_{1-6}$ -Alkanoyl ist, wenn A ein Rest $-CH_2SR^1$ ist.

Unter der Bezeichnung Retinoidcarbonsäuren sollen im folgenden Verbindungen verstanden werden, die den in der Retinsäure vorkommenden Nonatetraensäurerest oder den davon durch Cyclisierung abgeleiteten Carboxyphenyl-propen-2-yl--Rest enthalten, welche Reste auch substituiert sein können. Solche Retinoidcarbonsäuren sind z.B. in den US-PS 4 215 215, 4 054 589, 4 326 055 und 4 476 056 beschrieben. Von besonderem Interesse sind Retinoidcarbonsäuren der Formeln

$$Z^1-CH=CH-C(CH_3)=CH-CH=CH-C(CH_3)=CH-COOH \qquad (a)$$

und

$$Z^2-C(CH_3)=CH-Ph-COOH \qquad (b)$$

ist, wobei $Z^1$ ein substituierter Phenyl- oder Cyclohexenylrest, $Z^2$ ein substituierter bicyclisch carbocyclischer oder heterocyclischer Rest und Ph ein Phenylenrest ist und wobei die Doppelbindungen in der Polyenkette von (a) E- oder Z-Konfiguration aufweisen können.

Beispiele substituierter Phenylreste $Z^1$ sind Phenylreste, die durch $C_{1-6}$-Alkyl, $C_{1-6}$-Alkoxy, Halogen, oder Trifluormethoxy vorzugsweise mehrfach substituiert sind. Beispiele substituierter Cyclohexenylrest sind 2,6,6-Tri-methylcyclohexen-1-yl, 2,6,6-Trimethyl-3-hydroxycyclohexen--1-yl und 2,6,6-Trimethyl-3-oxocyclohexen-1-yl. Beispiele substituierter bicyclisch carbocyclischer Reste sind 1,1,3,3-Tetramethyl-5-indanyl und 5,6,7,8-Tetrahydro--5,5,8,8-tetramethyl-2-naphthyl, Beispiele bicyclisch heterocyclischer Reste sind 4,4-Dimethyl-6-chromanyl, 4,4-Dimethyl-6-thiochromanyl, 4,4-Dimethyl-1,2,3,4-tetra-hydro-6-chinolinyl und 4,4-Dimethyl-6-thiochromanyl-1,1--dioxid.

Bevorzugte Verbindungen der Formel I sind die, in denen Ret der Acylrest der all-trans-9-(4-Methoxy-2,3,6-trimethylphenyl)-3,7-dimethyl -2,4,6,8-nonatetraensäure, der p-[(E)-2-(5,6,7,8-Tetrahydro-5,5,8,8-tetramethyl -2-naphthyl)propenyl]benzoesäure, der all-trans-Vitamin-A-Säure oder der 13-cis-Vitamin-A-Säure ist.

Bevorzugte Reste A sind solche der Formel $-C(R^4)_2OC(O)R^3$, insbesondere diejenigen, in denen $R^3$ $C_{1-6}$-Alkyl und $R^4$ Wasserstoff oder $C_{1-6}$-Alkyl ist. Beispiele von $C_{1-6}$-Alkyl und $C_{1-6}$-Alkoxy sind geradkettige und verzweigte Alkylreste mit 1-6 C-Atomen wie Methyl, Aethyl, Propyl, Isopropyl, Butyl, tert.-Butyl, n-Pentyl und n-Hexyl. Falls in A zwei Reste $R^1$, $R^2$ oder $R^4$ vorhanden sind, können diese gleich oder voneinander verschieden sein.

Substituierte Phenylreste und Styrylreste $R^3$ können einen od rere Substituenten wie Hydroxy, $C_{1-6}$-Alkoxy, und $C_{1-6}$-Alkanoyloxy enthalten. Beispiele solcher Reste sind Hydroxyphenyl, 3,4,5-Trimethoxyphenyl, Acetoxyphenyl und Methoxystyryl. Ein 2-Benzoyl-phenoxy-$C_{1-6}$-alkylrest kann im Phenoxyteil durch C

$_{1-6}$-Alkyl, C $_{1-6}$-Alkoxy oder Halogen substituiert sein.

Die Verbindungen der Formel I können erfindungsgemäss dadurch hergestellt werden, dass man eine Retinoidcarbonsäure der Formel Ret-OH oder ein reaktionsfähiges Derivat davon mit einem Alkohol der Formel HA oder einem reaktionsfähigen Derivat davon oder einem Halogenid der Formel Hal-A, wobei Ret und A die oben angegebene Bedeutung haben und Hal Halogen ist, oder eine Verbindung der Formel Ret-O-C($R^4$)$_2$-Hal mit einer Carbonsäure der Formel $R^{31}$COOH, wobei Ret, Hal und $R^4$ die oben angegebene Bedeutung haben und $R^{31}$ C $_{1-6}$-Alkyl Phenyl, substituiertes Phenyl, Styryl oder im Phenylrest substituiertes Styryl ist, in Gegenwart eines Kondensationsmittels umsetzt und gegebenenfalls reaktionsfähige Gruppen im Reaktionsprodukt funktionell abwandelt.

In einer Ausführungsform des erfindungsgemässen Verfahrens setzt man eine Retinoidcarbonsäure der Forme Ret-OH mit einem Alkohol der Formel HA in Gegenwart eines Kondensationsmittels wie N,N'-Carbonyldiimidazol oder N,N'-Dicyclohexylcarbodiimid um. Die Umsetzung wird zweckmässig in einem inerten, organischen Lösungsmittel, z.B. einem Aether wie Tetrahydrofuran oder Dioxan vorgenommen. Die Reaktionstemperatur ist nicht kritisch, zweckmässig arbeitet man bei Raumtemperatur. Vorzugsweise wird die Umsetzung unter Inertgas und Ausschluss von Licht und Feuchtigkeit durchgeführt.

In einer anderen Ausführungsform des erfindungsgemässen Verfahrens kann ein reaktionsfähiges Derivat einer Retinoidcarbonsäure Ret-OH, z.B. ein Säurehalogenid oder -anhydrid, vorzugsweise das Säurechlorid, mit einem Alkohol der Formel HA umgesetzt werden. Als Kondensationsmittel für diese Umsetzung eignen sich Basen, wie Amin, z.B. Triäthylamin.

In einer weiteren Ausführungsform des erfindungsgemässen Verfahrens kann eine Retionidcarbonsäure Ret-OH mit einem reaktionsfähigen Derivat eines Alkohols HA umgesetzt werden. Als Beispiele reaktionsfähiger Derivate kommen z.B. Tosylate und Mesylate oder Halogenide in Betracht, d.h. Verbindungen in denen eine Hydroxygruppe durch eine Tosyloxy-oder Mesyloxygruppe oder durch Halogen, insbesondere Jod, ersetzt ist. Schliesslich kann ein Halogenalkylester einer Retinoidcarbonsäure, d.h. eine Verbindung Ret-O-C($R^4$)$_2$-Hal mit einer Carbonsäure $R^{31}$COOH umgesetzt werden. Ein bevorzugtes Kondensationsmittel für diese Umsetzungen ist das 1,8-Diazabicyclo(5,4,0)undec-7-en(1,5-5). Als Halogenalkylester ist ein Chloralkylester, insbesondere der Chlormethylester, bevorzugt, als Carbonsäure $R^{31}$COOH eine Alkancarbonsäure, wie Essigsäure. Alle diese Umsetzungen können in an sich bekannter Weise durchgeführt werden, z.B. wie in Tetrahedron, Vol. 36, 2409-2422, 1980, beschrieben. Vorzugsweise verwendet man in den vorstehend beschriebenen Reaktionen einen Alkohol HA, in dem nur eine freie Hydroxgryuppe vorliegt und allfällig vorhandene weitere Hydroxgruppen in geschützter Form, beispielsweise als leicht spaltbare Aether, wie Tetrahydropyranyläther, oder als leicht spaltbare Ester vorliegen. Solche Schutzgruppen werden aus dem primär erhaltenen Reaktionsprodukt des Alkohols HA mit der Retionoidcarbonsäure oder deren reaktiven Derivaten in an sich bekannter Weise abgespalten, um eine Verbindung der Formel I zu erhalten.

Die Reaktionsprodukte können in an sich bekannter Weise aufgearbeitet werden, z.B. durch Abdestillieren des Lösungsmittels, Verteilen der Bestandteile des Rückstandes zwischen einem organischen Lösungsmittel und einer wässrigen Phase und Chromatographie, z.B. an Silicagel und/oder Kristallisation aus einem Lösungsmittel.

Im Reaktionsprodukt der Formel I enthaltene reaktiv e Gruppen können in an sich bekannter Weise funktionell abgewandelt werden.

Beispielsweise können Hydroxygruppen und Mercaptogruppen durch Alkylierung oder Acylierung in C $_{1-6}$-Alkoxy- oder C $_{1-6}$-Alkanoyloxygruyppen bzw. C $_{1-6}$-Alkylthio oder C $_{1-6}$-Alkanoylthiogruppen umgewandelt werden. C $_{1-6}$-Alkylthiogruppen können durch Behandlung mit Oxidationsmitteln wie Persäuren in C $_{1-6}$-Alkylsulfinyl und C $_{1-6}$-Alkylsulfonylgruppen umgewandelt werden.

Die Verbindungen der Formel I sind therapeutisch wirksam und können zur vorzugsweise topischen Behandlung von Erkrankungen verwendet werden, die mit Verhornungsstörungen der Haut einhergehen, wie z.B. Psoriasis, Ichthyosis und Morbus Darier und bei Störungen der Fibroblastenaktivitäten, wie z.B. Keloidose und lokalisierte Sklerodermie; sowie bei Präkanzerosen der Haut und bei Akne.

Die Verbindungen zeichnen sich besonders durch eine gute Verträglichkeit, z.B. verminderte Hautirritationen bei der topischen Verabreichung, aus. Von besonderem Interesse ist das Methylenacetat (all-E)-9-(4-methoxy-2,3,6-trimethylphenyl)-3,7-dimethyl-2,4,6,8-nonate raenoat.

Die Wirksamkeit der Verbindungen kann an Mäusen geprüft, werden bei denen durch Behandlung mit Dimethylbenzanthracen und Krotonöl Papillome der Haut erzeugt wurden. Durch topische Verabreichung von Verbindungen der Formel I wird eine Rückbildung der Papillome beobachtet, die ein Mass für die therapeutische Wirksamkeit der Verbindungen, z.B. für die Behandlung der Psoriasis, darstellt. Die Versuchsmethodik zur Erzeugung der Papillome ist in Europ. J. Cancer, Vol. 10, 731-737 (1974) beschrieben. Die Therapie der Hautpapillome wird aufgenommen, wenn die Mäuse mindestens 6 Papillome mit einem Mindestdurchmesser von 5 mm entwickelt haben. Gruppen von jeweils 4 Tieren werden die Verbindungen

in einem geeigneten Vehikel auf jeweils 3 Papillome aufgetragen. Insgesamt finden 14 Applikationen von jeweils 2,5 µl einer Wirkstofflösung pro Papillom statt und zwar einmal täglich an den Tagen 2, 3, 4, 5, 8, 9, 10, 11, 12, 15, 16, 17, 18 und 19. 4 Tiere dienen als Kontrollgruppe, bei denen nur das Vehikel appliziert wird.

Zur Evaluierung der Wirksamkeit der Verbindungen wird die Summe der Durchmesser der 3 behandelten Papillome bestimmt und der Durchschnittswert für jede Gruppe berechnet. Die Messungen werden durchgeführt vor Beginn der Therapie (Tag 1) und an den Tagen 8, 15 und 22. Die Zunahme oder Abnahme der durchschnittlichen Summe der Papillomdurchmesser pro Tier wird ausgedrückt als Prozent des Ausgangswertes (Tag 1).

Bei diesen Versuchen wurde z.B. bei Applikation der in Beispiel 3 hergestellten Verbindung als gesättigte Lösung in Isopropylmyristat eine Regression der behandelten Papillome von 90% beobachtet. Bei Kontrolltieren bei denen die Papillome nur mit dem Vehikel (Isopropylmyristat) behandelt worden waren, zeigte sich praktisch keine Regression der Papillondurchmesser.

Die Verbindungen zeichnen sich durch eine gute Penetrationsfähigkeit in die Haut aus. Bei in-vitro Versuchen mit Haut von nackten Ratten wurde z.B. bei der Verbindung von Beispiel 3 eine 200-fach stärkere Pentration als bei der entsprechenden Retinoidcarbonsäure (all-E-9-(4-Methoxy-2,3,6-trimethylphenyl)-3,7-dimethyl -2,4,6,8-nonatetraensäure) beobachtet.

Zur topischen Anwendung werden die Wirkstoffe zweckmässig in Form von Salben, Tinkturen, Crèmen, Lösungen, Lotionen, Sprays, Suspensionen und dgl. verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig ca. 0,01-5%ige, vorzugsweise 0,05-1%ige Lösungen und Lotionen, sowie ca. 0,01-5%ige, vorzugsweise ca. 0,05-2%ige Salben oder Crèmen geeignet.

Den Präparaten kann gegebenenfalls ein Antioxydationsmittel, z.B. Tocopherol, N-Methyl-γ-tocopheramin sowie butyliertes Hydroxyanisol oder butyliertes Hydroxytoluol beigemischt sein. Weiterhin können die Präparate andere Wirkstoffe, insbesondere Strahlenschutzmittel wie Silikate, Talk, Titandioxid, Zinkoxid oder Zimtsäurederivate wie >Parsol< enthalten.

Die nachstehenden Beispiele erläutern die Erfindung weiter. Die Temperaturen sind in Celsiusgraden angegeben.

Beispiel 1

4.9 g all-E-9-(4-Methoxy-2,3,6-trimethylphenyl)-3,7-dimethyl -2,4,6,8-nonatetraensäure werden in 100 ml trockenem Tetrahydrofuran suspendiert, mit 2.9 g N,N-Carbonyldiimidazol versetzt und 4 Stunden unter Stickstoffbegasung, Licht- und Feuchtigkeitsausschluss gerührt. In einem zweiten Reaktionskolben werden 11.2 g Triethylenglykol in 40 ml trockenem Tetrahydrofuran mit 0.4 g Natriumhydrid-Dispersion (55-60% in Oel) versetzt. Nach Ende der Wasserstoffentwicklung werden die beiden Lösungen vereinigt und noch 4 Stunden bei Raumtemperatur gerührt. Dann zieht man das Lösungsmittel am Rotationsverdampfer im Vakuum ab, nimmt den öligen Rückstand in 300 ml Methylenchlorid auf, schüttelt einmal mit 150 ml verdünnter Salzsäurelösung und zweimal mit je 150 ml gesättigter Kochsalzlösung aus und trocknet die organische Phase über Natriumsulfat. Das nach Filtration und Eindampfen verbleibende Oel wird mittels Chromatographie über 250 g Kieselgel mit Methylenchlorid-tert.-Butylmethylether 7:3 gereinigt. Als erste Fraktion wird der als Nebenprodukt entstandene Diester abgetrennt (Schmelzpunkt: 128-129°C aus Methylenchlorid-Petrolether). Als Hauptfraktion eluiert 2-(2-Hydroxyäthoxy)äthoxy)äthyl(all-E)-9-(4-methoxy-2,3,6 -trimethylphenyl)-3,7-dimethyl-2,4,6,8-nonatetraenoat. Nach Eindampfen des Eluates im Hochvakuum verbleibt eine gelbe, wachsartige Masse. Ausbeute: 4.4 g, $R_F$ (Kieselgel/Methylenchlorid-tert.Butylmethylether 7:3): 0.36.

Beispiel 2

In Analogie zu Beispiel 1 erhält man aus all-E-9-(4Methoxy-2,3,6-trimethylphenyl)-3,7-dimethyl -2,4,6,8-nonatetraensäure und Tetraäthylenglykol nach Aufarbeitung des Reaktionsgemisches (Chromatographie über Kieselgel mit Methylenchlorid-tert.Butylmethyläther 7:3 als Elutionsmittel) neben dem Diester das 2-(2-(2-(2-Hydroxyäthoxy)äthoxy)äthyl)äthyl (all-E)-9-(4-methoxy-2,3,6-trimethylphenyl)-3,7-dimethyl -2,4,6,8-nonatetraenoat als zähflüssiges Oel. $R_F$ (Kieselgel/Methylenchlorid-tert.Butylmethyläther 7:3): 0.23

Beispiel 3

Das Produkt aus Beispiel 2 lässt sich auch durch Acylierung des Alkohols mit dem Säurechlorid gewinnen. Dazu werden 16,3 g all-E-9-(4-Methoxy-2,3,6-trimethylphenyl)-3,7-dimethyl -2,4,6,8-nonatetraensäure in 200 ml trockenem Toluol suspendiert und mit 4.7 g Phosphortrichlorid versetzt. Das Reaktionsgemisch wird 15 h unter Stickstoffbegasung, Licht- und Feuchtigkeitsausschluss bei Raumtemperatur gerührt. Die entstandene Säurechloridlösung wird einer mit Eis auf 0°C gekühlten Lösungs aus 48 g Tetraäthylenglykol und 13 g Triäthylamin in 350 ml Methylenchlorid tropfenweise zugesetzt. Nach Ende der Zugabe wird noch 3 Stunden bei Raumtemperatur nachgerührt. Die Reaktionslösung giesst man in 500 ml gesättigte Kochsalzlösung. Die organische Phase trennt man ab, schüttelt noch zweimal mit jeweils 200 ml gesättigter Kochsalzlösung aus und trocknet die organische Phase über Natriumsulfat. Die gelbe Lösung wird zur Entfernung der noch vorhandenen Säure über 50 g Florisil filtriert, im Vakuum eingeengt, und der Rückstand durch Chromatographie über 350 g Kieselgel mit Methylenchlorid-tert.-Butylmethyläther 7:3 als Laufmittel gereinigt. Die das Produkt enthaltenden Fraktionen werden vereinigt und im Hochvakuum vom Lösungsmittel befreit. Es verbleiben 16.5 g eines gelben Oeles, welches mit dem Produk Beispiel 2 identisch ist.

Beispiel 4

Die Herstellung des Esters aus Beispiel 2 gelingt auch durch Alkylierung der Säure mit einem Tetraäthylenglykolderivat, bei welchem eine Hydroxylgruppe durch eine reaktive Abgangsgruppe ersetzt ist. Dazu werden 4.9 g all-E-9-(4-Methoxy-2,3,6-trimethylphenyl)-3,7-dimethyl -2,4,6,8-nonatetraensäure in 80 ml trockenem Acetonitril suspendiert und nach Zugabe von 2,3 g 1,8-Diazabicyclo(5,4,0)undec-7-en(1,5-5) bei Raumtemperatur unter Stickstoffbegasung, Licht- und Feuchtigkeitsausschluss 30 Minuten gerührt. Dann werden 5.2 g Tetraäthylenglykol-monotosylat und 0.5 g Natriumjodid zugesetzt und man rührt 15 Stunden bei Raumtemperatur. Das Lösungsmittel wird im Vakuum abgedampft, der Rückstand in 250 ml Methylenchlorid aufgenommen, einmal mit 100 ml 2 N Salzsuaure und zweimal mit jeweils 100 ml gesättigter Kochsalzlösung ausgeschüttelt. Die organische Phase wird über Natriumsulfat getrocknet und zur Entfernung von nicht umgesetzter Säure über 50 g Florisil filtriert. Der nach dem Abziehen des Lösemittels verbleibende Rückstand wird über 150 g Kieselgel mit Methylenchlorid-tert.Butylmethyläther (3:2) als Eluens chromatographiert. Es können 4.2 g eines gelben Oeles isoliert werden, welches mit dem Produkt aus Beispiel 2 identisch ist.

Das als Ausgangsmaterial verwendete Tetraäthylenglykol-monotosylat wurde wie folgt hergestellt:

Eine 0°C kalte Lösung aus 23.3 g Tetraäthylenglykol und 19 g Pyridin in 100 ml Methylenchlorid wird unter kräftigem Rühren portionsweise mit insgesamt 19 g p-Toluolsulfonsäurechlorid versetzt. Nach Beendigung der Zugabe rührt man noch 5 Stunden bei Raumtemperatur weiter. Dann gibt man 200 ml Methylenchlorid und 100 ml Wasser zu und trennt die organische Phase ab. Man wäscht nacheinander mit 100 ml 2 N Salzsäure, 100 ml 5%-iger Natriumhydrogencarbonatlösung und 100 ml Wasser und trocknet über Natriumsulfat. Das Lösungsmittel wird im Vakuum abgezogen und der Rückstand über 400 g Kieselgel mit Methylenglykol-Aethylacetat-Methanol (5:4:1) als Eluens chromatographiert. Neben geringen Mengen Tetraäthylenglykol-ditosylat werden 17 g Tetraäthylenglykol-monotosylat als farblose Flüssigkeit isoliert. $R_F$ (Kieselgel/Methylenchlorid-Ethylacetat-Methanol 5:4:1): 0.6

Beispiel 5

In Analogie zu Beispiel 1 lässt sich aus 3,7-Dimethyl-9-(2,6,6-trimethyl-1-cyclohexen-1-yl) -2(Z),4,6,8(E)-nonatetraensäure und Tetraäthylenglykol das 2(2-(2-(2-Hydroxyäthoxy)äthoxy)äthxoy)äthyl-3,7-dimethyl -9-(2,6,6-trimethyl-1-cyclohexen-1-yl) -2(Z),4,6,8(E)-nonatetraenoat herstellen. Nach Chromatographie über Kieselgel mit Methylchlorid-tert.Butylmethyläther (3:2) als Eluationsmittel und Abdampfen des Lösungsmittels im Hochvakuum fällt der Ester als gelbes, viskoses Oel an. $R_F$ (Kieselgel/Methylenchlorid-tert.Butylmethyläther 3:2): 0.43

Beispiel 6

In Analogie zu Beispiel 1 erhält man aus all-E-3,7Dimethyl-9-(2,6,6-trimethyl-1-cyclohexen-1-yl) -2,4,6,8nonatetraensäure und Tetraäthylenglykol nach Chromatographie des Reaktionsgemisches (Kiegelgel/Methylenchlorid-tert.Butylmethyläther 3:2) das 2-(2-(2-(2-Hydroxyäthoxy)äthoxy)äthoxy)äthyl (all-E)-3,7-dimethyl-9-(2,6,6-trimethyl-1-cyclohexen-1-yl) -2,4,6,8-nonatetraenoat als gelbes, zähflüssiges Oel. $R_F$ (Kieselgel/Methylenchlorid-tert.Butylmethyläther 3:2); 0.36.

Beispiel 7

In Analogie zu Beispiel 1 lässt sicht aus all-E-9-(4Methoxy-2,3,6-trimethylphenyl)-3,7-dimethyl -2,4,6,8-nonatetraensäure und Polyäthylenglykol 400 das Polyäthylenglykol(400)-mono-(all-E)-9-(4-methoxy-2,3,6 -trimethylphenyl)-3,7-dimethyl-2,4,6,8-nonatetraenoat herstellen. Das Reaktionsgemisch wird durch Chromatographie über Kieselgel gereinigt. Dabei wird zunächst mit Methylenchlorid-Aethylacetat (1:1) der in geringer Menge entstandene Diester eluiert. Der Monoester kann dann mit Aceton-Aethylacetat (1:1) ausgewaschen werden. M an erhält ein Polyethylenglykolestergemisch mit einer mittleren Aethylenoxidzahl von 9. Das Produkt ist eine gelbe, Viskosität Flüssigkeit, $R_F$ (Kieselgel/Aethylacetat-Aceton 1:1): 0.1-0.6 (mehrere Flecken); $R_F$ (HPTLC-RP-18/Methnaol): 0.55

Beispiel 8

In Analogie zu Beispiel 1 lässt sich aus all-E-9-(4-Methoxy-2,3,6-trimethylphenyl)-3,7-dimethyl -2,4,6,8-nonatetraensäure und Polyäthylenglykol 600 das Polyäthylenglykol(600)-mono-(all-E)-9-(4-methoxy-2,3,6 -trimethylphenyl)3,7-dimethyl-2,4,6,8-nonatetraenoat herstellen. Das Reaktionsgemisch wird durch Chromatographie über Kieselgel gereinigt. Zunächst wird mit Aethylacetat der in geringer Menge mitentstandene Diester abgetrennt. Der Monoester kann mit Aethylacetat-Aceton (1:2) eluiert werden. Nach Abziehen des Lösungsmittels im Hochvakuum verbleibt ein gelbes, viskoses Oel, welches analytisch als Polyäthylenglykolestergemisch mit einer mittleren Aethylenoxidzahl von 13 identifiziert wird. $R_F$ (Kieselgel/Aethylacetat-Aceton 1:2): 0.1-0.55 (mehrere Flecken); $R_F$ (HPTLC-RP-18/Methanol): 0.57

Beispiel 9

In Analogie zu Beispiel 1 erhält man aus all-E-9-(4-Methoxy-2,3,6-trimethylphenyl)-3,7-dimethyl -2,4,6,8-nonatetraensäure und Polyäthylenglykol 1000 das Polyäthyenglykol(1000)-mono-(all-E)-9-(4-methoxy-2,3,6 -trimethylphenyl)-3,7-dimethy-2,4,6,8-nonatetraenoat. Das Produkt wird durch Chromatographie über Kieselgel mit Methylenchlorid-Methanol 9-1 als Eluationsmittel gereinigt. Man erhält nach Abziehen des Lösemittels ein gelbes Wachs mit Schmelzpunkt 32-34° C, welches als Polyäthylenglykolestergemisch mit einer mittleren Aethylenoxidzahl von 22 identifiziert wird. $R_F$(Kieselgel/Methylenchlorid-Methanol 9:1): 0.5; $R_F$-(HPTLC-RP-18/Methanol-Wasser 9:1): 0.21

Beispiel 10

In Analogie zu Beispiel 1 lässt sich aus all-E-9-(4-Methoxy-2,3,6-trimethylphenyl)-3,7-dimethyl -2,4,6,8-nonatetraensäure und 2-Methylthio-äthanol das 2-(Methylthio)äthyl (all-E)-9-(4-methoxy-2,3,6-trimethylphenyl)-3,7-dimethyl -2,4,6,8-nonatetraenoat herstellen. Zur Isolierung des Produktes aus dem Reaktionsgemisch wird extrahiert und über Kieselgel mit Methylenchlorid als Eluationsmittel chromatographiert. Schmelzpunkt: 104° C (aus Methylenchlorid-Hexan), $R_F$ (Kieselgel/Methylenchlorid): 0.63

Beispiel 11

Eine eisgekühlte Lösung aus 10 g 2-(Methylthio)äthyl (all-E)-9-(4-methoxy-2,3,6-trimethylphenyl)-3,7-dimethyl -2,4,6,8-nonatetraenoat (Herstellung in Beispiel 10) in 220 ml Methylenchlorid wird innerhalb von 60 Minuten portionsweise mit insgesamt 4.5 g 3-Chlorperbenzoesäure versetzt. Die Reaktionslösung wird noch 2 Stunden bei 0° C nachgerührt. Man gibt weitere 100 ml Methylenchlorid zu und schüttelt zweimal mit jeweils 100 ml einer 5%-igen Natriumhydrogencarbonatlösung und zweimal mit je 100 ml Wasser aus. Nach Filtration über 50 g Florisil chromatographiert man den eingedampften Rückstand über 250 g

Kieselgel mit Methylenchlorid-tert.Butylmethyläther (2:3) als Eluationsmittel. Die Produkt enthaltenden Fraktionen werden vereinigt, im Vakuum eingedampft und aus Methylenchlorid-Hexan umkristallisiert. Man erhält 5.1 g 2-(Methylsulfinyl)äthyl (all-E)-9-(4-methoxy-2,3,6-trimethylphenyl)-3,7-dimethyl -2,4,6,8-nonatetraenoat. Schmelzpunkt: 109-110° C, R $_F$ (Kieselgel/Methylenchlorid-tert.-Butylmethyläther 2:3): 0.17

Beispiel 12

In Analogie zu Beispiel 1 erhält man aus all-E-9-(4-Methoxy-2,3,6-trimethylphenyl)-3,7-dimethyl -2,4,6,8-nonatetraensäure und 2-Hydroxyäthyl-methyl-sulfon das 2-(Methylsulfonyl)äthyl (all-E)-9-(4-methoxy-2,3,6-trimethylphenyl)-3,7-dimethyl -2,4,6,8-nonatetraenoat. Das Produkt wird nach Filtration über Florisil aus Methylenchlorid-Hexan umkristallisiert. Schmelzpunkt: 128-129° C, R $_F$ (Kieselgel/Methylenchlorid-tert.Butylmethyläther 9:1): 0 .74

Beispiel 13

In Analogie zu Beispiel 1 lässt sich aus all-E-9-(4-Methoxy-2,3,6-trimethylphenyl)-3,7-dimethyl -2,4,6,8-nonatetraensäure und 1-(2-Hydroxyäthyl)-azacyclopentan-2-on das 2-(2-Oxo-1-pyrrolidinyl)äthyl (all-E-)-9-(4-methoxy-2,3,6-trimethylphenyl)-3,7-dimethyl -2,4,6,8-nonateraenoat herstellen. Das Produkt kann man durch Chromatographie über Kieselgel mit Methylenchlorid-Aethylester (1:1) isolieren. Schmelzpunkt: 83-84° C (aus Methylchlorid-Hexan), R $_F$(Methylenchlorid-Aethylacetat 1:1): 0.35

Beispiel 14

In Analogie zu Beispiel 1 wird aus all-E-3,7-Dimethyl-9-(2,6,6-trimethyl-1-cyclohexen-1-yl) 2,4,6,8-nonatetraensäure und 1-(2-Hydroxyäthyl)-azacyclopentan-2-on das 2-(2-Oxo-1-pyrrolidinyl)äthyl (all-E)-9-(2,6,6-trimethyl-1-cyclohexen-1-yl)-3,7-dimethyl -2,4,6,8-nonatetraenoat erhalten. Nach Chromatographie über Kieselgel mit Methylenchlorid-Aethylacetat (7:3) und Kristallisation aus Methylenchlorid-Petroläther erhält man gelbe Kristalle mit SchmelxPunkt 64-66° C. R $_F$ (Kieselgel/Methylenchlorid-Aethylacetat 7:3): 0.30

Beispiel 15

Analog zu Beispiel 1 erhält man aus 3,7-Dimethyl-9-(2,6,6-trimethyl-1-cyclohexen-1-yl) -2(Z),4,6,8(E)-nonatetraensäure und 1-(2-Hydroxyäthyl)-azacyclopentan-2-on das 2-(2-Oxo-1-pyrrolidinyl)äthyl-3,7-dimethyl-9-(2,6,6-trimethyl -1-cyclohexen-1-yl)-2(Z),4,6,8(E)-nonatetraenoat. Das Produkt wird nach Chromatographie über Kieselgel mit Methylenchlorid-Aethylacetat (7:3) aus Methylchlorid-Petroläther umkristallisiert. Man erhält gelbe Kristalle mit einem Schmelzpunkt von 87-89° C. R $_F$ (Kieselgel/Methylenchlorid-Aethylacetat 7:3): 0.41

Beispiel 16

In Analogie zu Beispiel 1 wird aus all-E-9-(4-Methoxy-2,3,6-trimethylphenyl)-3,7-dimethyl -2,4,6,8-nonatetraensäure und 1-(2-Hydroxyäthyl)-succinimid das 2-Succinimidoäthyl all-E-9-(4-Methoxy-2,3,6-trimethylphenyl)-3,7-dimethyl -2,4,6,8-nonatetraenoat hergestellt. Zur Aufarbeitung des Reaktionsgemisches wird nach der Extraktion über Kieselgel mit Methylenchlorid-Aethylacetat (4:1) chromatographiert und aus Methylenchlorid-Hexan umkristallisiert. Man erhält gelbe Kristalle mit einem Schmelzpunkt von 127° C. R $_F$ -(Kieselgel/Methylenchlorid-Aethylacetat 4:1): 0.65

Beispiel 17

In Analogie zu Beispiel 1 wird aus all-E-9-(4-Methoxy-2,3,6-trimethylphenyl)-3,7-dimethyl -2,4,6,8-nonatetraensäure und 1-(2-Hydroxyäthyl)-glutarimid das 2-Glutarimidoethyl (all-E)-9-(4-methoxy-2,3,6-trimethylphenyl)-3,7-dimethyl -2,4,6,8-nonatetraenoat hergestellt. Das Produkt wird durch Chromatographie über Kieselgel mit Methylenchlorid-Aethylacetat (9:1) und Kristallisation aus Methylenchlorid-Hexan isoliert. Man erhält gelbe Kristalle mit einem Schmelzpunkt von 127-129 °C. $R_F$ (Kieselgel/Methylenchlorid-Aethylacetat 9:1): 0.5

Beispiel 18

Analog zu Beispiel 1 erhält man aus all-E-9-(4-Methoxy-2,3,6-trimethylphenyl)-3,7-dimethyl -2,4,6,8-nonatetraensäure und 1-(2-Hydroxyäthyl)-piperidin das 2-(1-Piperidyl)ethyl (all-E)-9-(4-methoxy-2,3,6-trimethylphenyl)-3,7-dimethyl -2,4,6,8-nonatetraenoat.Der Ester wird mit Methylenchlorid-Methanol (2:1) über Kieselgel chromatographiert und aus Petroläther umkristallisiert. Schmelzpunkt: 52-53 °C. $R_R$ - (Kieselgel/Methylenchlorid-Methanol 2:1): 0.21.

Beispiel 19

In Analogie zu Beispiel 1 wird aus all-E-9-(4-Methoxy-2,3,6-trimethylphenyl)-3,7-dimethyl -2,4,6,8-nonatetraensäure und 4-(2-Hydroxyäthyl)-morpholin das 2-(4-Morpholinyl)ethyl all-E-9-(4-methoxy-2,3,6-trimethylphenyl)-3,7-dimethyl -2,4,6,8-nonatetraenoat hergestellt. Das Produkt wird aus Methylenchlorid-Hexan umkristallisiert. Schmelzpunkt: 93 °C, $R_F$ (Kieselgel/Methylenchlorid-tert.Butylmethyläther 4:1): 0.23.

Beispiel 20

In Analogi e zu Beispiel 1 wird aus all-E-9-(4-Methoxy-2,3,6-trimethylphenyl)-3,7-dimethyl -2,4,6,8-nonatetraensäure und N,N-Diäthyl-hydroxylamin das N,N-Diäthyl-O--[(all-E)-9-(4-methoxy-2,3,6-trimethylphenyl) -3,7-dimethyl-2,4,6,8-nonatetraenoyl]hydroxylamin (Schmelzpunkt: 85-86 °C aus Petroläther, $R_F$ - (Kieselgel/Cyclohexan-Aethylacetat 7-3): 0.44) und das N,N-Diäthyl-O-9-[(2Z,4E,6E,8E)(4-methoxy-2,3,6 - trimethylphenyl)-3,7-dimethyl-2,4,6,8 -nonatetraenoyl]hydroxylamin (Schmelzpunkt: 138-140 °C aus Petroläther, $R_F$ (Kieselgel/Cyclohexan-Aethylacetat 7:3): 0.52) hergestellt. Die beiden Isomeren werden durch Chromatographie über Kieselgel mit Methylenchlorid-tert.Butylmethyläther 95:5 getrennt.

Beispiel 21

Zu einer Suspension aus 3.2 g all-E-9-(4-Methoxy-2,3,6-trimethylphenyl)-3,7-dimethyl -2,4,6,8-nonatetraensäure in 70 ml trockenem Acetonitril gibt man 1.6 g 1,8-Diazabicyclo(5,4,0)undec-7-en(1,5-5) und rührt 1 Stunde bei Raumtemperatur unter Stickstoffbegasung, Licht- und Feuchtigkeitsausschluss. Dann setzt man 3 g Jodmethylacetat in 40 ml Acetonitril zu und rührt 15 Stunden bei Raumtemperatur. Das Lösemittel wird im Vakuum abgezogenen, der Rückstand in 200 ml Methylenchlorid aufgenommen, einmal mit 80 ml 2N Salzsäure und zweimal mit je 100 ml gesättigter Kochsalzlösung gewaschen. Die organische Phase trocknet man über Natriumsulfat. Zur Entfernung der nicht umgesetzten Säure filtriert man über 40 g Florisil, wäscht mit Methylenglycol-Aether (1:1) nach und chromatographiert das Filtrat über 150 g Kieselgel mit insgesamt 1.3 l Methylenchlorid-Hexan (3:1). Das Eluat wird eingedampft, das verbleibende gelbe Oel aus Methylenchlorid-Petroläther umkristallisiert. Man erhält 2.3 g Methylenacetat (all-E)-9-(4-methoxy-2,3,6-trimethylphenyl)3,7-dimethyl -2,4,6,8-nonatetraenoat als orangegelbe Kristalle mit einem Schmelzpunkt von 78-79 °C. $R_F$ (Kieselgel/Hexan-Methylenchloird-tert.-Butylmethyläther 50:45:5): 0.59

Das als Ausgangsmaterial verwendete Jodmethylacetat wird wie folgt hergestellt:

21.7 g Chlormethylacetat (hergestellt nach L.H.Ulich, R.Adams:J.Am.Chem.Soc. 43 , 660-667 (1921)) und 45 g Natriumjodid in 200 ml trockenem Aceton rührt man 2 Stunden bei 40 °C. Nach Abkühlen trennt man vom ausgefallenen Natriumchlorid ab, dampft das Filtrat ein, nimmt den Rückstand in 200 ml Methylenchlorid auf und schüttelt zweimal mit je 100 ml 5%-iger Natriumsulfitlösung und zweimal mit je 100 ml Wasser aus. Die organische Phase wird über Natriumsulfat getrocknet, das Lösemittel abgezogen und der Rückstand unter Zusatz einer Spatelspitze Natriumsulfat unter Lichtausschluss im Wasserstrahlvakuum destilliert. Man erhält eine farblose Flüssigkeit mit Siedepunkt 49-51 °C/13 mmHg.

Beispiel 22

In Analogie zu Beispiel 21 erhält man aus all-E-9-(4-Methoxy-2,3,6-trimethylphenyl)-3,7-dimethyl-2,4,6,8-nonatetraensäure und Jodmethylpivalat des Methylen (all-E)-9-(4-methoxy-2,3,6-trimethylphenyl)-3,7-dimethyl -2,4,6,8-nonatetraenoat pivalat. Nach Kristallisation aus Petroläther fallen gelbe Kristalle mit einem Schmelzpunkt von 109-111 °C an. $R_F$ (Kieselgel/Hexan-Methylchlorid-tert.Butylmethyläther 50:45:5): 0.6

Beispiel 23

Analog zu Beispiel 21 wird aus all-E-3,7-Dimethyl-9-(2,6,6-trimethyl-1-cyclohexen-1-yl) -2,4,6,8-nonatetraensäure und Jodmethylpivalat das Methylen (all-E)-3,7-dimethyl-9-(2,6,6-trimethyl-1-cyclohexen-1-yl) -2,4,6,8-nonatetraenoat pivalat hergestellt. Das Produkt wird nach Extraktion des Reaktionsgemisches über Kieselgel mit Methylenchlorid-Cyclohexan (1:1) chromatographisiert. Man erhält nach Kristallisation aus Methanol gelbe Kristalle mit einem Schmelzpunkt von 61-62 °C. $R_F$ (Kieselgel/Methylenchlorid-Cyclohexan 1:1): 0.42.

Beispiel 24

In Analogie zu Beispiel 21 erhält man aus all-E-9-(4-Methoxy-2,3,6-trimethylphenyl)-3,7-dimeth yl -2,4,6,8-nonatetraensäure und Chlormethylbenzoat in Anwesenheit von Natriumjodid das Methylen benzoat (all-E)-9-(4-methoxy-2,3,6-trimethylphenyl)-3,7-dimethyl -2,4,6,8-nonatetraenoat.Zur Isolierung des Produktes aus dem Reaktionsgemisch wird nach der Extraktion über Kieselgel mit Methylenchlorid-Hexan (7:3) chromatographiert. Nach Kristallisation aus Methylenchlorid-Petroläther fällt der Ester in gelben Kristallen mit einem Schmelzpunkt von 136-139 °C an. $R_F$(Kieselgel/Hexan-Methylenchlorid-tert.Butylmethyläther 50:45:5): 0.74

Beispiel 25

Analog zu Beispiel 21 lässt sich aus all-E-9-(4-Methoxy-2,3,6-trimethylphenyl)-3,7-dimethyl -2,4,6,8-nonatetraensäure und Jodmethyl 3,4,5-trimethoxybenzoat das Methylen (all-E)-9-(4-methoxy-2,3,6-trimethylphenyl)-3,7dimethyl -2,4,6,8-nonatetraenoat 3,4,5-trimethoxybenzoat herstellen. Nach der Extraktion des Reaktionsgemisches wird die organische Phase über Florisil filtriert, mit Methylenchlorid-Aether (1:1) nachgewaschen und im Vakuum eingeengt. Der Rückstand kristallisiert aus Aethylacetat-Petroläther mit einem Schmelzpunkt von 106-108 °C. $R_F$ (Kieselgel/Cyclohexan-Aethylacetat 7:3): 0.53.

Das als Ausgangsmaterial verwendete Jodmethyl 3,4,5-trimethoxybenzoat wird auf folgende Art hergestell:

Zu einer gerührten Mischung aus 21.1 g 3,4,5-Trimethoxybenzoesäure, 33.6 g Natriumhydrogencarbonat und 3.4 g Tetrabutylamonium hydrogensulfat in 600 ml Methylenchlorid-Wasser (1:1) werden bei Raumtemperatur tropfenweise 19 g Chormethyl-chlorsulfat in 30 ml Methylenchlorid eingetragen. Zur Vervollständigung der Reaktion rührt man noch 2 Stunden bei Raumtemperatur nach, trennt dann die beiden Phasen, wäscht die wässrige Phase mit 100 ml Methylenchlorid und trocknet die vereinigten organischen Phasen über Natriumsulfat. Das Lösungsmittel wird im Vakuum abgezogen, der Rückstand über 450 gl Kieselgel mit Methylenchlorid chromatographiert. Die Produkt enthaltenden Fraktionen werden eingedampft, der Rückstand aus Methylenchlorid-Petroläther kristallisiert. Man erhält 10.5 g Chlormethyl 3,4,5-trimethoxybenzoat als farblose Nadeln mit einem Schmelzpunkt von 85-86 °C. $R_F$ - (Kieselgel/Methylenchlorid): 0.45.

7.8 g Chormethyl 3,4,5-trimethoxybenzoat werden in 150 ml Acetonitril gelöst, mit 9 g Natriumjodid versetzt und 15 Stunden bei Raumtemperatur unter Stickstoffbegasung und Lichtausschluss gerührt. Dann wird vom ausgefallenen Natriumchlorid abfiltriert und das Filtrat im Vakuum eingedampft. Den Rückstand nimmt man in 150 ml Aether auf, schüttelt einmal mit 100 ml einer 5%-igen Natriumsulfitlösung und zweimal mit je 100 ml Wasser und trocknet die organische Phase über Natriumsulfat. Nach Filtern des Trockenmittels wird der Aether im Vakuum abgezogen und der ölige Rückstand ohne weitere Reinigung für die weitere Umsetzung eingesetzt. $R_F$ (Kieselgel/Methylenchlorid-Petroläther 4:1): 0.45

Beispiel 26

In Analogie zu Beispiel 21 wird aus all-E-9-(4-Methoxy2,3,6-trimethylphenyl)-3,7-dimethyl -2,4,6,8-nonatetraensäure und Jodmethyl o-acetoxybenzoat das Methylen o-acetoxybenzoat (all-E)-9-(4-methoxy-2,3,6-trimethylphenyl)-3,7-dimethyl -2,4,6,8-nonatetraenoat hergestellt. Schmelzpunkt: 97-98 °C (aus Aethylacetat-Petroläther), $R_F$ (Kieselgel/Cyclohexan-Aethylacetat 7:3): 0.57

Das als Ausgangsmaterial verwendete Jodmethyl o-acetoxybenzoat wurde aus Acetylsalicylsäure und Chlormethyl chlorsulfat wird in Beispiel 25 für das Chlormethyl 3,4,5-trimethoxybenzoat beschrieben hergestellt. Das Chlormethyl o-acetoxybenzoat (farblose Flüssigkeit, $R_F$ (Kieselgel/Methylenchlorid): 0.68) wird mit Natriumjodid in das Jodmethyl o-acetoxybenzoat (gelbliches Oel, $R_F$ (Kieselgel/Methylenchlorid): 0.7) übergeführt.

Beispiel 27

Analog zu Beispiel 21 lässt sich aus all-E-9-(4-Methoxy-2,3,6-trimethylphenyl)-3,7-dimethyl -2,4,6,8-tetrano naensäure und Jodmethyl [(2-benzoyl-5-methoxy)phenoxy]-acetat das Methylen [(2-benzoyl-5-methoxy)phenoxy]-acetat (all-E)-9-(4-methoxy-2,3,6-trimethylphenyl)-3,7-dimethyl -2,4,6,8-nonatetraenoat herstellen. Zur Reinigung des Reaktionsproduktes wird über Kieselgel mit Methylenchlorid-Petroläther-tert.Butylmethyläther (26:10:1) chromatographiert und aus Aethylacetat-Petroläther umkristallisiert. Man erhält gelbe Kristalle mit einem Schmelzpunkt von 119-121 °C. $R_F$ (Kieselgel/Methylenchlorid-Petroläther-tert. Butylmethyläther 26:10:1): 0.5

Das als Ausgangsmaterial verwendete Jodmethyl [(2-benzoyl-5-methoxy)phenoxy-acetat wird in Analogie zum Jodmethyl 3,4,5-trimethoxybenzoat aus Beispiel 25 aus 2-(2-Benzoyl-5-methoxy)phenoxy-essigsäure und Chlormethylchlorsulfat hergestellt. Die Umwandlung des Chlormethyl [(2-benzoyl-5-methoxy)-phenxoy]acetat (farbloser Feststoff, Schmelzpunkt: 70-72 °C aus Methylenchlorid-Petroläther) zum Jodmethyl [(2-benzoyl-5-methoxy)phenoxy]-acetat erfolgt mit Natriumjodid in Acetonitril. $R_F$ -(Kieselgel/Methylenchlorid): 0.44

Beispiel 28

In Analogie zu Beispiel 21 wird aus all-E-9-(4-Methoxy-2,3,6-trimethylphenyl)-3,7-dimethyl, 2,4,6,8-nonatetraensäure und Jodmethyl-cinnamat das Methylen cinnamat (all-E)-9-(4-methoxy-2,3,6-trimethylphenyl)-3,7-dimethyl -2,4,6,8-nonatetraenoat hergestellt. Nach Kristallisation aus Petroläther erhält man gelbe Kristalle mit einem Schmelzpunkt von 67-69 °C. $R_F$ (Kieselgel/Cyclohexan-Aethylacetat 7:3): 0.66

Das als Ausgangsmaterial verwendete Jodmethylcinnamat wird aus Zimtsäure und Chlormethylchlorsulfat wie für das Jodmethyl 3,4,5-trimethoxybenzoat in Beispiel 25 beschrieben hergestellt. Das Chlormethylcinnamat (gelbliche Flüssigkeit, $R_F$ (Kieselgel/Methylenchlorid-Petroläther 4:1): 0.74) wird mit Natriumjodid in das Jodmethylencinnamat umgewandelt. $R_F$ (Kieselgel/ Methylenchlorid-Petroläther 4:1): 0.78.

Beispiel 29

Analog zu Beispiel 21 wird aus all-E-9-(4-Methoxy-2,3,6-trimethylphenyl)-3,7-dimethyl -2,4,6,8-nonatetraensäure und Jodmethyl-p-methoxycinnamat das Methylen p-methoxycinnamat (all-E)-9-(4-methoxy-2,3,6-trimethylphenyl)-3,7-dimethyl -2,4,6,8-nonatetraenoat hergestellt. Schmelzpunkt: 86-88 °C (aus Aethanol). $R_F$ (Kieselgel/Cyclohexan-Aethylacetat 8:2): 0.4

Das als Ausgangsprodukt verwendete Jodmethyl-p-methoxycinnamat wird in Analogie zu Jodmethyl-3,4,5-trimethoxybenzoat aus Beispiel 25 aus p-Methoxyzimt-säure und Chlormethyl-chlorsulfat hergestellt. Das so erhaltene Chlormethyl p-methoxycinnamat (farbloser Feststoff mit Schmelzpunkt 64-65 °C aus Aethylacetat-Petroläther) wird mit Natriumjodid in Acetonitril in das Jodmethyl-p-methoxycinnamat umgewandelt. Man erhält ein gelbliches Wachs mit $R_F$ (Kieselgel/Cyclohexan-Aethylacetat 8:2): 0.52

Beispiel 30

13.1 g all-E-9-(4-Methoxy-2,3,6-trimethylphenyl)-3,7-dimethy-2,4,6,8-nonatetraensäure und 5.6 g fein

gepulvertes Kaliumcarbonat werden in 250 ml Dimethylformamid während 2 h bei 80 °C gerührt. Nach Zugabe von 5.5 g 1-Chloräthylacetat in 20 ml Dimethylformamid und 1 g Kaliumiodid wird das Reaktionsgemisch 18 h bei 80 °C gerührt. Danach wird das Lösungsmittel am Rotationsverdampfer im Vakuum abgezogen, der Rückstand in 300 ml Methylenchlorid aufgenommen, einmal mit 150 ml verdünnter Salzsäurelösung und zweimal mit 150 ml gesättigter Natriumchloridlösung ausgeschüttelt. Die Methylenchloridphase trocknet man über Natriumsulfat und filtriert über 80 g Florisil. Das Florisil wäscht man mit Methylenchlorid-tert. Butylmethyläther 95:5 nach. Das nach Abziehen des Lösungsmittels verbleibende gelbe Oel wird gereinigt durch Chromatographieren über 300 g Kieselgel mit Methylenchlorid/Petroläther/tert.Butylmethyläther 30:65:5 als Elutionsmittel. Die reinen Fraktionen werden vereinigt, im Vakuum eingeengt und der Rückstand kristallisiert. Man er hält 6.6 g Aethylidenacetat (all-E)-9-(4-methoxy-2,3,6-trimethylphenyl)-3,7-dimethyl-2,4,6,8-nonatetraenoat als gelbe Kristalle mit einem Schmelzpunkt von 106-108 °C (Methylenchlorid-Petrolether). $R_F$ (Kieselgel/Methylenchlorid-Petrolether-tert.Butylmethylether 30:65:5) = 0.34.

Beispiel 31

Analog zu Beispiel 30 erhält man aus all-E-9-(4-Methoxy-2,3,6-trimethylphenyl)-3,7-dimethyl-2,4,6,8-nonatetraensäure und 1-Chloräthylbenzoat das Aethylidenbenzoat (all-E)-9-(4-methoxy-2,3,6-trimethylphenyl)-3,7-dimethyl-2,4,6,8-nonatetraenoat.Die Reinigung nach Ausschütteln und Florisilfiltration erfolgt durch zweimalige Kristallisation aus Methylenchlorid-Methanol. Man erhält gelbe Kristalle mit einem Schmelzpunkt von 123-125 °C. $R_F$ (Kieselgel/Methylenchlorid-Hexan-tert.-Butylmethylether 30:65:5) = 0.4

Beispiel 32

In Analogie zu Beispiel 30 wird aus all-E-9-(4-Methoxy-2,3,6-trimethylphenyl)-3,7-dimethyl-2,4,6,8-nonatetraensäure und 1-Chloräthylcarbonat in Acetonitril als Solvens das Aethyliden (äthylcarbonat) (all-E)-9-(4-methoxy-2,3,6-trimethylphenyl)-3,7-dimethyl-2,4,6,8-nonate raenoat hergestellt. Nach Ausschütteln und Florisilfiltration wird aus Aceton-Methanol kristallisiert. Man erhält gelbe Kristalle mit einem Schmelzpunkt von 117-118 °C. $R_F$ (Methylenchlorid-Petrolether 3:1) = 0.71.

Beispiel 33

Das Produkt aus Beispiel 21 lässt sich auch durch Alkylierung der Essigsäure mit dem Chlormethylester der Retinsäure herstellen. Dazu werden 0.16 g Essigsäure und 0.41 g DBU (1,8-Diazabicyclo(5,4,0)-undec-7-en(1,5-5)) in 20 ml trockenem Acetonitril 30 min gerührt. Diese Lösung versetzt man mit 0.94 g Chloromethyl (all-E)-9-(4-methoxy-2,3,6-trimethylphenyl)-3,7-dimethyl-2,4,6,8-nonate raenoat und rührt das Reaktionsgemisch unter Licht-und Feuchtigkeitsausschluss 2 h bei 80 °C. Das Lösungsmittel wird am Rotationsverdampfer im Vakuum abgezogen, der Rückstand in 50 ml Methylenchlorid aufgenommen, mit 25 ml verdünnter Salzsäurelösung und zweimal mit 25 ml gesättigter Natriumchloridlösung ausgeschüttelt. Die organische Phase trocknet man über Natriumsulfat und filtriert über 15 g Florisil. Nach Eindampfen des Lösungsmittels kristallisiert man das verbleibende Oel aus 10 ml Aethanol. Man erhält 0.86 g orangegelbe Kristalle mit einem Schmelzpunkt von 78-80 °C, welche mit dem Produkt aus Beispiel 21 identisch sind.

Das als Ausgangsprodukt verwendete Chlormethyl (all-E)-9-(4-methoxy-2,3,6-trimethylphenyl)-3,7-dimethyl-2,4,6,8-nonate raenoat wurde auf folgende Weise hergestellt:

Zu einer gerührten Mischung aus 6.5 g all-E-9-(4-Methoxy-2,3,6-trimethylphenyl)-3,7-dimethyl-2,4,6,8-nonatetraensäure, 0.68 g Tetrabutylammoniumhydrogensulfat und 6.7 g Natriumhydrogencarbonat in 80 ml Wasser/150 ml Methylenchlorid werden bei Raumtemperatur tropfenweise 4.3 g Chlormethylchlorsulfat in 25 ml Methylenchlorid gegeben. Nach Ende der Zugabe wird das Reaktionsgemisch noch 2 h nachgerührt. Die Phasen werden getrennt, die wässrige Phase wird nochmals mit 50 ml Methylenchlorid ausgeschüttelt und die vereinten organischen Phasen werden über Natriumsulfat getrocknet. Zur Entfernung von nicht umgesetztem Ausgangsprodukt filtriert man über 50 g Florisil und wäscht mit Methylenchlorid nach. Nach Abdampfen des Lösungsmittels chromatographiert man das verbleibende Oel auf 250 g Kieselgel mit Methylenchlorid-Petroläther 1:1 als Laufmittel. Die das Produkt enthaltenden Fraktionen werden vereinigt und im Vakuum vom Lösungsmittel befreit. Der ölige Rückstand wird aus Methylenchlorid/Petroläther kristallisiert. Man erhält gelbe Kristalle (5.3 g) mit einem Schmelzpunkt von 106-108 °C. $R_F$ -

(Kieselgel/Methylenchlorid-Petrolether 1:1) = 0.38.

## Beispiel A (Gel)

| | |
|---|---|
| Wirkstoff | 0.05 - 1 g |
| Hydroxypropylcellulose | 2 - 5 g |
| Propylenglykol | 5 - 20 g |
| Ethanol | 40 - 80 g |
| Wasser | ad 100 g |

Der Wirkstoff wird in Ethanol klar gelöst. Nach Beimischen der Propylenglykol-Wasser-Lösung lässt man die Hydroxypropylcellulose klar ausquellen.

## Beispiel B (Crème, Typ O/W)

| | |
|---|---|
| Wirkstoff | 0.05 - 2 g |
| Polyoxyethylen-sorbitanester | 3 - 10 g |
| Cetylalkohol | 7 - 12 g |
| Vaseline, weiss | 15 - 35 g |
| Glycerin | 5 - 15 g |
| Benzoesäure | 0.1 - 0.3 g |
| Wasser | ad 100 g |

Bei ca 70-75 °C wird der Wirkstoff in die geschmolzene Fettphase eingearbeitet. Der wässrigen Benzoesäurelösung werden Glycerin und der Emulgator beigemischt. Die Wasser-und die Fettphase werden bei ca. 70 °C homogenisiert und unter Homogenisieren auf Raumtemperatur erkalten gelassen.

## Beispiel C (Crème, Typ W/O)

| | |
|---|---|
| Wirkstoff | 0.05 - 2 g |
| Vaseline, weiss | 20 - 40 g |
| Wachs, weiss | 5 - 15 g |
| Paraffinöl, dickflüssig | 10 - 20 g |
| Glycerinsorbitanfettsäureester | 5 - 10 g |
| Benzoesäure | 0.1 - 0.2 g |
| Wasser | ad 100 g |

In die geschmolzene Fettphase wird bei ca. 80 °C der Wirkstoff eingearbeitet. Die ebenfalls ca. 80 °C heisse, wässrige Benzoesäurelösung wird unter Homogenisieren der Fettphase beigegeben und die

Emulsion unter weiterem Homogenisieren auf Raumtemperatur erkalten gelassen.

**Beispiel D (Salbe)**

| | |
|---|---|
| Wirkstoff | 0.05 - 2 g |
| Paraffin, dickflüssig | 30 - 50 g |
| Vaseline, weiss | 40 - 50 g |
| Ricinusöl, gehärtet | ad 100 g |

In die ca 80° C heisse Fettphase arbeitet man den Wirkstoff ein und lässt das Gemisch unter Rühren auf Raumtemperatur abkühlen.

## Ansprüche

1. Verbindungen der Formel

$$\text{Ret-OA} \qquad \text{I}$$

worin

- Ret der Acylrest einer Retinoidcarbonsäure, A ein Rest $(-CHR-CH_2O)nR^1$, $(-CH_2)mSR^1$, $(-CH_2)_m XR^2$, $(-CH_2)_m$Het, $-N(R^2)_2$, $-C(R^4)_2OC(O)R^3$, $-CH_2-CH(OR^2)CH_2OR^2$ oder $-CH(CH_2OR^2)_2$;
- R Wasserstoff oder Methyl,
- $R^1$ Wasserstoff, $C_{1-6}$-Alkyl oder $C_{1-6}$-Alkanoyl,
- $R^2$ $C_{1-6}$-Alkyl,
- $R^3$ $C_{1-6}$-Alkyl, $C_{1-6}$-Alkoxy, Di-$C_{1-6}$-Alkylamino, Phenyl, substituiertes Phenyl, Styryl oder im Phenylrest substituiertes Styryl oder 2-Benzoyl-phenoxy--$C_{1-6}$-alkyl;
- $R^4$ Wasserstoff, $C_{1-6}$-Alkyl oder Phenyl;
- X >SO oder $>SO_2$,
- Het Pyrrolidino, Piperidino, Mono- oder Diketopyrrolidino, Mono-oder Diketopiperidino, Piperazino, $N^4$-$C_{1-4}$-Alkylpiperazino, Morpholino oder Thiamorpholino,
- n eine ganze Zahl von 3-40 und
- m eine ganze Zahl von 1-4 ist, wobei $R^1$ $C_{1-6}$-Alkyl oder $C_{1-6}$-Alkanoyl ist, wenn A ein Rest $-CH_2SR^1$ ist.

2. Verbindungen gemäss Anspruch 1, in denen Ret der Acylrest einer Retinoidcarbonsäure der Formel

$$Z^1-CH{=}CH-C(CH_3){=}CH-CH{=}CH-C(CH_3){=}CH-COOH \qquad (a)$$

und

$$Z^2-C(CH_3){=}CH-Ph-COOH \qquad (b)$$

ist, wobei $Z^1$ ein substituierter Phenyl- oder Cyclohexenylrest und $Z^2$ ein substituierter bicyclisch carbocyclischer oder heterocyclischer Rest und Ph ein Phenylenrest ist und wobei die Doppelbindungen in der Polyenkette von (a) E- oder Z-Konfiguration aufweisen können.

3. Verbindungen gemäss Anspruch 2, in denen Ret der Acylrest der all-trans-9-(4-Methoxy-2,3,6-trimethylphenyl)--3,7-dimethyl -2,4,6,8-nonatetraensäure, der p-[(E)-2--(5,6,7,8-Tetrahydro-5,5,8,8-tetramethyl -2-naphthyl)pro-penyl]benzoesäure, der all-trans-Vitamin-A-Säure oder der 13-cis-Vitamin-A-Säure ist.

4. Verbindungen gemäss den Ansprüchen 1-3, in denen A ein Rest $-C(R^4)_2OC(O)R^3$ ist.

5. Verbindungen gemäss Anspruch 4, in denen $R^3$ $C_{1-6}$-Alkyl und $R^4$ Wasserstoff oder $C_{1-6}$ -Alkyl ist.

6. Methylenacetat (all-E)-9-(4-methoxy-2,3,6-trimethyl-phenyl)-3,7-dimethyl-2,4,6,8-nonatetraenoat.

7. 2-(2-(2-(2-Hydroxyäthoxy)äthoxy)äthoxy)äthyl (all-E)-9-(4-methoxy-2,3,6-trimethylphenyl)-3,7-dimethyl-2,4,6,8-nonatetraenoat,
2-(2-(HYdroxyäthoxy)äthoxy)äthyl(all-E)-9-(4-methoxy-2,3,6-trimethylphenyl)-3,7-dimethyl-2,4,6,8-nonatetraenoat,
2-(2-(2-(2-Hydroxyäthoxy)äthoxy)äthoxy)äthyl-3,7-dimethyl-9-(2,6,6-trimethyl-2-cyclohexen-1-yl)-2-(Z),4, 6,8(E)-nonatetraenoat,
2-(2-(2-(2-Hydroxyäthoxy)äthoxy)äthoxy)äthyl (all-E)-3,7-dimethyl-9-(2,6,6-trimethyl-1-cyclohexen-1-yl)-2,4,6,8-nonatetraenoat,
Polyäthylenglykol(400)-mono-(all-E)-9-(4-methoxy-2,3,6-trimethylphenyl)-3,7-dimethyl-2,4,6,8-nonatetraenoat,
Polyäthylenglykol(6OO)-mono-(all-E)-9-(4-methoxy-2,3,6-trimethylphenyl)-3,7-dimethyl-2,4, 6, 8-nonatetraenoat,
Polyäthylenglykol(1000)-mono-(all-E)-9-(4-methoxy-2,3,6-trimethylphenyl)-3,7-dimethyl-2,4, 6, 8-nonatetraenoat,
2-(Methylthio)äthyl (all-E)-9-(4-methoxy-2,3,6-trimethylphenyl)-3,7-dimethyl-2,4,6,8-nonatetraenoat,
2-(Methylsulfinyl)äthyl (all-E)-9-(4-methoxy-2,3,6-trimethylphenyl)-3,7-dimethyl-2,4,6,8-nonate raenoat,
2-(Methylsulfonyl)äthyl (all-E)-9-(4-methoxy-2,3,6-trimethylphenyl)-3,7-dimethyl-2,4,6,8-nonate raenoat,
2-(2-Oxo-1-pyrrolidinyl)äthyl (all-E)-9-(4-methoxy-2,3,6-trimethylphenyl)-3,7-dimethyl-2,4,6,8-nonate raenoat,
2-(2-Oxo-1-pyrrolidinyl)äthyl (all-E)-9-(2,6,6-trimethyl-1-cyclohexen-1-yl)-3,7-dimethyl-2,4,6,8-nona etraenoat,
2-(2-Oxo-1-pyrrolidinyl)äthyl-3,7-dimeth yl-9-(2,6,6-trimethyl-1-cyclohexen-1-yl)-2(Z),4,6,8-(E)-nonatetraenoat,
2-Succinimidoäthyl all-E-9-(4-Methoxy-2,3,6-trimethylphenyl)-3,7-dimethyl-2,4,6,8-nonatetr enoat,
2-Glutarimidoäthyl (all-E)-9-(4-methoxy-2,3,6-trimethylphenyl)-3,7-dimethyl-2,4,6,8-nonate raenoat,
2-(1-Pirperidyl)äthyl (all-E)-9-(4-methoxy-2,3,6-trimethylphenyl)-3,7-dimethyl-2,4,6,8-nonate raenoat,
2-Morpholinyl)ethyl all-E-9-(4-methoxy-2,3,6-trimethylphenyl)-3,7-dimethyl-2,4,6,8-nonatetr enoat,
N,N-Diäthyl-O-[(all-E)-9-(4-methoxy-2,3,6-tr imethylphenyl)-3,7-dimethyl-2,4,6,8-nonatetraenoyl]-hydroxylamin,
N,N -Diäthyl-O-9-[(2Z,4E,6E,8E)(4-methoxy-2,3,6,-trimethylp henyl)-3,7-dimethyl-2,4,6,8-nonatetraenoyl]hydroxylamin,
Methylen (all-E)-9-(4-methoxy-2,3,6-trimethylphenyl)-3,7-dimethyl-2,4,6,8-nonate raenoat pivalat,
Methylen (all-E)-3,7-dimethyl-9-(2,6,6,-trimethyl-1-cyclohexen-1-yl)-2,4,6,8-non tetraenoat pivalat,
Methylen benzoat (all-E)-9-(4-methoxy-2,3,6-trimethylphenyl)-3,7-dimethyl-2,4,6,8-nonate raenoat,
Methylen (all-E)-9-(4-methoxy-2,3,6-trimethylphenyl)-3,7-dimethyl-2,4,6,8-nonate raenoat 3,4,5,-trimethoxybenzoat,
Methylen o-acetoxybenzoat (all-E)-9-(4-methoxy-2,3,6-trimethylphenyl)-3,7-dimethyl-2,4,6,8-nonatetraenoat,
Methylen [(2-benzoyl-4-methoxy)phenoxy]-acetat (all-E)-9-(4-methoxy-2,3,6-trimethylphenyl)-3,7-dimethyl-2,4,6,8-nonate raenoat,
Methylen-cinnamat (all-E)-9-(4-methoxy-2,3,6-trimethylphenyl)-3,7-dimethyl-2,4,6,8-nonate raenoat,
Methylen-p-methoxycinnamat (all-E)-9-(4-methoxy-2,3,6-trimethylphenyl)-3,7-dimethyl-2,4,6,8-nonate raenoat,
Aethyliden-(äthylcarbonat) (all-E)-9-(4-methoxy-2,3,6-trimethylphenyl)-3,7-dimethyl-2,4,6,8-nonate raenoat,
Aethylidenbenzoat-(all-E)-9-(4-methoxy-2,3 ,6-trimethylphenyl)-3,7-dimethyl-2,4,6,8-nonatetraenoat,

Aethylidenacetat (all-E)-9-(4-methoxy-2,3,6-trimethylphenyl)-3,7-dimethyl-2,4,6,8-nonate raneoat.

**8.** Die Verbindungen der Ansprüche 1-7 als Heilmittel.

**9.** Verwendung der Verbindungen der Ansprüche 1-7 zur Herstellung von Heilmitteln zur Behandlung von dermatologischen Erkrankungen.

**10.** Pharmazeutische Präparate enthaltend eine Verbindung der Ansprüche 1-7 als Wirkstoff und übliche pharmazeutische Trägerstoffe.

**11.** Verfahren zur Herstellung der Verbindungen von Anspruch 1, dadurch gekennzeichnet, dass man eine Retinoidcarbonsäure der Formel Ret-OH oder ein reaktionsfähiges Derivat davon mit einem Alkohol der Formel HA oder einem reaktionsfähigen Derivat davon, oder einem Halogenid der Formel Hal-A, wobei Ret und A die in Anspruch 1 angegebene Bedeutung haben und Hal Halogen ist, oder eine Verbindung der Formel Ret-O-C($R^4$)$_2$-Hal mit einer Carbonsäure der Formel $R^{31}$COOH, wobei Ret, Hal und $R^4$ die oben angegebene Bedeutung haben und $R^{31}$ $C_{1-6}$-Alkyl, Phenyl, substituiertes Phenyl, Styryl oder im Phenylrest substituiertes Styryl ist, in Gegenwart eines Kondensationsmittels umsetzt und gegebenenfalls reaktionsfähige Gruppe im Reaktionsprodukt funktionell abwandelt.

## Claims

**1.** Compounds of the formula

$$\text{Ret-OA} \qquad \text{I}$$

wherein

| | |
|---|---|
| Ret | is the acyl residue of a retinoid carboxylic acid, A is a residue $(-CHR-CH_2O)_nR^1$, $(-CH_2)_mSR^1$, $(-CH_2)_mXR^2$, $(-CH_2)$m-Het, $-N(R^{2)}{}_2$, $(-CH_2)_mXR^2$, $(-CH_2)_m$ -Het, $-N(R^2)_2$, $-C(R^4)_2OC(O)R^3$, $-CH_2-CK(OR^2)CH_2OR^2$ or $-CH(CH_2OR^2)_2$; |
| R | is hydrogen or methyl, |
| $R^1$ | is hydrogen, $C_{1-6}$ -alkyl or $C_{1-6}$ -alkanoyl, |
| $R^2$ | is $C_{1-6}$-alkyl, |
| $R_3$ | is $C_{1-6}$ -alkyl, $C_{1-6}$alkoxy, di-$C_{1-6}$ -alkylamino, $R^3$ is $C_{1-6}$ -alkyl, $C_{1-6}$-alkoxy, di-$C_{1-6}$-alkylamino, phenyl, substituted phenyl, styryl or styryl or 2-benzoyl-phenoxy-$C_{1-6}$-alkyl substituted in the phenyl residue; |
| $R^4$ | is hydrogen, $C_{1-6}$-alkyl or phenyl; |
| x | is >SO or >$SO_2$, |
| Het | is pyrrolidino, piperidino, mono- or diketopyrrolidino, mono- or diketopiperidino, piperazino, $N^4$-$C_{1-4}$-alkylpiperazino, morpholino or thiamorpholino, |
| n | is a whole number of 3-40 and |
| m | is a whole number of 1-4, whereby $R^1$ is $C_{1-6}$-alkyl or $C_{1-6}$-alkanoyl when A is a $-CH_2SR^1$ residue. |

**2.** Compounds in accordance with claim 1, in which Ret is the acyl residue of a retinoid carboxylic acid of the formula

$$Z^1\text{-CH=CH-C(CH}_3\text{)=CH-CH=CH-C(CH}_3\text{)=CH-COOH} \qquad (a)$$

and

$$Z^2\text{-C(CH}_3\text{)=CH-Ph-COOH} \qquad (b)$$

wherein $Z^1$ is a substituted phenyl or cyclohexenyl residue and $Z^2$ is a substituted bicyclic carbocyclic

or heterocyclic residue and Ph is a phenylene residue and wherein the double bonds in the polyene chain of (a) can have the E- or Z-configuration.

3. Compounds in accordance with claim 2, in which Ret is the acyl residue of all-trans-9-(4-methoxy-2,3,6--trimethylphenyl)-3,7-dimethyl-2,4,6,8-nonatetraenoic acid, of p-[(E)-2-(5,6,7,8-tetrahydro-5,5,8,8-tetramethyl--2-naphthyl)propenyl]benzoic acid, of all-trans-vitamin-A acid or of 13-cis-vitamin-A acid.

4. Compounds in accordance with claims 1-3, in which A is a residue $-C(R^4)_2OC(O)R^3$.

5. Compounds in accordance with claim 4, in which $R^3$ is $C_{1-6}$-alkyl and $R^4$ is hydrogen or $C_{1-6}$-alkyl.

6. Methylene acetate (all-E)-9-(4-methoxy-2,3,6--trimethylphenyl)-3,7-dimethyl-2,4,6,8-nonatetraenoate.

7. 2-(2-(2-(2-Hydroxyethoxy)ethoxy)ethoxy)ethyl (all-E)-9-(4-methoxy-2,3,6-trimethylphenyl)-3,7-dimethyl--2,4,6,8-nonatetraenoate,
2-(2-(hydroxyethoxy)ethoxy)ethyl (all-E)-9-(4-methoxy--2,3,6-trimethylphenyl)-3,7-dimethyl-2,4,6,8-nonatetra-enoate,
2-(2-(2-(2-hydroxyethoxy)ethoxy)ethoxy)ethyl 3,7--dimethyl-9-(2,6,6-trimethyl-2-cyclohexen-1-yl)-2-(Z),4,6. 8(E)-nonatetraenoate,
2-(2-(2-(2-hydroxyethoxy)ethoxy)ethoxy)ethyl (all-E)-3,7-dimethyl-9-(2,6,6-trimethyl-1-cyclohexen-1-yl)--2,4,6,8-nonatetraenoate,
polyethylene glycol (400) mono-(all-E)-9-(4-methoxy--2,3,6-trimethylphenyl)-3,7-dimethyl-2,4,6,8-nonatetra-enoate,
polyethylene glycol (600) mono-(all-E)-9-(4-methoxy--2,3,6-trimethylphenyl)-3,7-dimethyl-2,4,6,8-nonatetra-enoate,
polyethylene glycol (1000) mono-(all-E)-9-(4-methoxy--2,3,6-trimethylphenyl)-3,7-dimethyl-2,4,6,8-nonatetra-enoate,
2-(methylthio)ethyl (all-E)-9-(4-methoxy-2,3,6--trimethylphenyl)-3,7-dimethyl-2,4,6,8-nonatetraenoate,
2-(methylsulphinyl)ethyl (all-E)-9-(4-methoxy-2,3,6--trimethylphenyl)-3,7-dimethyl-2,4,6,8-nonatetraenoate,
2-(methylsulphonyl)ethyl (all-E)-9-(4-methoxy-2,3,6--trimethylphenyl)-3,7-dimethyl-2,4,6,8-nonatetraenoate,
2-(2-oxo-1-pyrrolidinyl)ethyl (all-E)-9-(4-methoxy--2,3,6-trimethylphenyl)-3,7-dimethyl-2,4,6,8-nonatetra-enoate,
2-(2-oxo-1-pyrrolidinyl)ethyl (all-E)-9-(2,6,6--trimethyl-1-cyclohexen-1-yl)-3,7-dimethyl-2,4,6,8-nonatetraenoate,
2-(2-oxo-1-pyrrolidinyl)ethyl 3,7-dimethyl-9-(2,6,6--trimethyl-1-cyclohexen-1-yl)-2(Z),4,6,8(E)-nonatetraenoate
2-succinimidoethyl all-E-9-(4-methoxy-2,3,6-trimethyl-phenyl)-3,7-dimethyl-2,4,6,8-nonatetraenoate,
2-glutarimidoethyl (all-E)-9-(4-methoxy-2,3,6--trimethylphenyl)-3,7-dimethyl-2,4,6,8-nonatetraenoate,
2-(1-piperidyl)ethyl (all-E)-9-(4-methoxy-2,3,6--trimethylphenyl)-3,7-dimethyl-2,4,6,8-nonatetraenoate,
2-morpholinyl)ethyl all-E-9-(4-methoxy-2,3,6--trimethylphenyl)-3,7-dimethyl-2,4,6,8-nonatetraenoate,
N,N-diethyl-O[(allE)-9-(4-methoxy-2,3,6-trimethyl-phenyl)-3,7-dimethyl-2,4,6,8-nonatetraenoyl]-hydroxylamine,
N,N-diethyl-O-9-[(2Z,4E,6E,8E)(4-methoxy-2,3,6--trimethylphenyl)-3,7-dimethyl-2,4,6,8-nonatetraenoyl]-hydroxylamine,
methylene (all-E)-9-(4-methoxy-2,3,6-trimethylphenyl)--3,7-dimethyl-2,4,6,8-nonatetraenoate pivalate,
methylene (all-E)-3,7-dimethyl-9-(2,6,6-trimethyl-1--cyclohexen-1-yl)-2,4,6,8-nonatetraenoate pivalate,
methylene benzoate (all-E)-9-(4-methoxy-2,3,6--trimethylphenyl)-3,7-dimethyl-2,4,6,8-nonatetraenoate,
methylene (all-E)-9-(4-methoxy-2,3,6-trimethylphenyl)--3,7-dimethyl-2,4,6,8-nonatetraenoate 3,4,5-trimethoxy-benzoate,
methylene o-acetoxybenzoate (all-E)-9-(4-methoxy--2-3,6-trimethylphenyl)-3,7-dimethyl-2,4,6,8-nonatetra-enoate,
methylene [(2-benzoyl-4-methoxy)phenoxy]-acetate (all-E)-9-(4-methoxy-2,3,6-trimethylphenyl)-3,7-dimethyl--2,4,6,8-nonatetraenoate,
methylene cinnamate (all-E)-9-(4-methoxy-2,3,6--trimethylphenyl)-3,7-dimethyl-2,4,6,8-nonatetraenoate,
methylene p-methoxycinnamate (all-E)-9-(4-methoxy--2,3,6-trimethylphenyl)-3,7-dimethyl-2,4,6,8-nonatetra-enoate,
ethylidene (ethyl carbonate) (all-E)-9-(4-methoxy--2,3,6-trimethylphenyl)-3,7-dimethyl-2,4,6,8-nonatetra-enoate-
ethylidene benzoate (all-E)-9-(4-methoxy-2,3,6--trimethylphenyl)-3,7-dimethyl-2,4,6,8-nonatetraenoate,
ethylidene acetate (all-E)-9-(4-methoxy-2,3,6--trimethylphenyl)-3,7-dimethyl-2,4,6,8-nonatetraenoate.

**8.** The compounds of claims 1-7 as medicaments.

**9.** The use of the compounds of claims 1-7 for the manufacture of medicaments for the treatment of dermatological disorders.

**10.** Pharmaceutical preparations containing a compound of claims 1-7 as the active substance and customary pharmaceutical carrier materials.

**11.** A process for the manufacture of the compounds of claim 1, characterized by reacting a retinoid carboxylic acid of the formula Ret-OH or a reactive derivative thereof with an alcohol of the formula HA or a reactive derivative thereof or a halide of the formula Hal-A, wherein Ret and A have the significance given in claim 1 and Hal is halogen, or reacting a compound of the formula Ret-O-C($R^4$)$_2$-Hal with a carboxylic acid of the formula $R^{31}$COOH, wherein Ret, Hal and $R^4$ have the significance given above and $R^{31}$ is $C_{1-6}$-alkyl, phenyl, substituted phenyl, styryl or styryl substituted in the phenyl residue, in the presence of a condensation agent and, if desired, functionally modifying reactive groups in the reaction product.

## Revendications

**1.** Composés de formule :

$$\text{Ret-OA} \qquad \text{I}$$

dans laquelle :

- Ret représente le radical acyle d'un acide carboxylique de rétinoide, A représente un radical --($CHR-CH_2O)_nR'_1$, $(-CH_2)_mSR^1$, $(-CH_2)_mXR^2$, $(-CH_2)_m$Het, $-N(R^2)_2$, $-C(R^4)_2OC(O)R^3$, $-CH_2-CH(OR^2)CH_2OR^2$ ou $-CH(CH_2OR^2)_2$ ;
- R représente l'hydrogène ou un groupe méthyle ;
- $R^1$ représente l'hydrogène, un groupe alkyle en $C_1$-$C_6$ ou alcanoyle en $C_1$-$C_6$,
- $R^2$ représente un groupe alkyle en $C_1$-$C_6$ ;
- $R^3$ représente un groupe alkyle en $C_1$-$C_6$, alcoxy en $C_1$-$C_6$, di-(alkyle en $C_1$-$C_6$)-amino, phényle, phényle substitué, styryle ou styryle substitué dans le noyau phényle, ou 2-benzoylphénoxyalkyle en $C_1$-$C_6$ ;
- $R^4$ représente l'hydrogène, un groupe alkyle en $C_1$-$C_6$ ou phényle,
- X représente un groupe >SO ou >$SO_2$,
- Het représente un groupe pyrrolidino, pipéridino, monoou dicétopyrrolidino, mono- ou dicétopipéridino, pipérazino, $N^4$-(alkyle en $C_1$-$C_4$)-pipérazino, morpholino ou thiamorpholino,
- n est un nombre entier allant de 3 à 40, et
- m est un nombre entier allant de I à 4,

sous réserve que $R^1$ représente un groupe alkyle en $C_1$-$C_6$ ou alcanoyle en $C_1$-$C_6$ lorsque A représente un radical $-CH_2SR^1$.

**2.** Composés selon la revendication 1, pour lesquels Ret représente le radical acyle d'un acide carboxylique de rétinoîde de formule :

$$Z^1\text{-CH=CH-C(CH}_3\text{)=CH-CH=CH-C(CH}_3\text{)=CH-COOH} \quad (a)$$

et

$$Z^2\text{-C(CH}_3\text{)=CH-Ph-COOH} \quad (b)$$

dans lesquelles $Z^1$ représente un groupe phényle ou cyclohexényle substitué et $Z^2$ représente un radical carbocyclique ou hétérocyclique bicyclique substitué et pH un groupe phénylène, les doubles liaisons de la chaîne polyénique de (a) pouvant être en configuration E ou Z.

3. Composés selon la revendication 2, pour lesquels Ret représente le radical acyle de l'acide tout-trans9-(4-méthoxy-2,3,6-triméthylphényl)-3,7-diméthyl-2,4,6,8-nonatétraénoîque, de l'acide p-[(E)-2-(5,6,7,8-tétrahy-dro-5,5,8,8-tétraméthyl-2-naphtyl)-propényl]-benzoîque, de l'acide tout-trans de la vitamine A ou de l'acide 13-cis de la vitamine A.

4. Composés selon les revendications 1 à 3, pour lesquels A représente un radical $-C(R^4)_2OC(O)R^3$.

5. Composés selon la revendication 4, pour lesquels $R^3$ représente un groupe alkyle en $C_1$-$C_6$ et $R^4$ l'hydrogène ou un groupe alkyle en $C_1$-$C_6$.

6. Le méthylène acétate (tout-E)-9-(4-méthoxy-2,3,6-triméthylphényl)-3,7-diméthyl-2,4,6,8-nonatétraénoate.

7. Le (tout-E)-9-(4-méthoxy-2,3,6-triméthylphényl)-3,7-diméthyl-2,4,6,8-nonatétraénoate de 2-(2-(2-hydroxy-éthoxy)-éthoxy) -éthoxy)-éthyle,
le (tout-E)-9-(4-méthoxy-2,3,6-triméthylphényl)-3,7-diméthyl-2,4,6,8-nonatétraénoate de 2-(2-(hydroxy-éthoxy)-éthoxy)-éthyle,
le 3,7-diméthYl-9-(2,6,6-triméthyl-2-cyclohexène-1-yl)-2(Z),4,6,8(E)-nonatétraénoate de 2-(2-(2-(2-hydroxy-éthoxy)-éthoxy)-éthoxy)-éthyle,
le (tout-E)-3,7-diméthyl-9-(2,6,6-triméthyl-l-cyclo-hexène-l-yl)-2,4,6,8-nonatétraénoate de 2-(2-(2-(2-hydroxyéthoxy)-éthoxy)-éthoxy)-éthyle,
le mono-(tout-E)-9-(4-méthoxy-2,3,6-triméthylphényl)-3,7-diméthyl-2,4,6,8-nonatétraénoate du polyéthylèneglycol (400),
le mono-(tout-E)-9-(4-méthoxy-2,3,6-triméthlylphényl)-3,7-diméthÿl-2,4,6,8-nonatétraénoate du polyéthylèneglycol (600),
le mono-(tout-E)-9-(4-méthoxy-2,3,6-triméthylphényl)-3,7-diméthyl-2,4,6,8-nonatétraénoate du polyéthylèneglycol (1000),
le (tout-E)-9-(4-méthoxy-2,3,6-triméthylphényl)-3,7-diméthyl-2,4,6,8-nonatétraénoate de 2-(méthylthio)-éthyle,
le (tout-E)-9-(4-méthoxy-2,3,6-triméthylphényl)-3,7-diméthyl-2,4,6,8-nonatétraénoate de 2-(méthylsulfinyl)-éthyle,
le (tout-E)-9-(4-méthoxy-2,3,6-triméthyphényl)-3,7-diméthyl-2,4,6,8-nonatétraénoate de 2-(méthylsulfonyl)-éthyle,
le (tout-E)-9-(4-méthoxy-2,3,6-triméthylphényl)-3,7-diméthyl-2,4,6,8-nonatétraénoate de 2-(2-oxo-l-pyr-rolidinyl)-éthyle,
le (tout-E)-9-(2,6,6-triméthyl-l-cyclohexène-l-yl)-3,7-diméthyl-2,4,6,8-nonatétraénoate de 2-(2-oxo-l-pyrrolidinyl)-éthyle,
le 3,7-diméthyl-9-(2,6,6-triméthyl-l-cyclohexène-l-yl)-2(Z),4,6,8(E)-nonatétraénoate de 2-(2-oxo-l-pyr-rolidinyl)-éthyle,
le (tout-E)-9-(4-méthoxy-2,3,6-triméthylphényl)-3,7-diméthyl-2,4,6,8-nonatétraénoate de 2-succinimido-éthyle,
le (tout-E)-9-(4-méthoxy-2,3,6-triméthylphényl)-3,7-diméthyl-2,4,6,8-nonatétraénoate de 2-glutarimido-éthyle,
le (tout-E)-9-(4-méthoxy-2,3,6-triméthylphényl)-3,7-diméthyl-2,4,6,8-nonatétraénoate de 2-(l-pipéridyl)-éthyle,
le (tout-E)-9-(4-méthoxy-2,3,6-triméthylphényl)-3,7-diméthyl-2,4,6,8-nonatétraénoate de 2-(morpholinyl)-éthyle,
la N,N-diéthyl-O-[(tout-E)-9-(4-méthoxy-2,3,6-triméthyl-phényl)-3,7-diméthyl-2,4,6,8-nonatétraénoyl]-hydroxylamine,
la N,N-diéthyl-O-9-[(2Z,4E,6E,8E)-(4-méthoxy-2,3,6-triméthylphényl)-3,7-diméthyl-2,4,6,8-nonatétraénoyl]-hydroxylamine,
le méthylène (tout-E)-9-(4-méthoxy-2,3,6-triméthyl-phényl)-3,7-diméthyl-2,4,6,8-nonatétraénoate pivalate,
le méthylène (tout-E)-3,7-diméthyl-9-(2,6,6-triméthyl-l-cyclohexène-l-yl)-2,4,6,8-nonatétraénoate pivalate,
le méthylène benzoate (tout-E)-9-(4-méthoxy-2,3,6-tri-méthylphényl)-3,7-diméthyl-2,4,6,8-nonatétraénoate,
le méthylène (tout-E)-9-(4-méthoxy-2,3,6-triméthyl-phényl)-3,7-diméthyl-2,4,6,8-nonatétraénoate 3,4,5-triméthoxybenzoate,
le méthylène o-acétoxybenzoate (tout-E)-9-(4-méthoxy-2,3,6-triméthylphényl)-3,7-diméthyl-2,4,6,8-nonaté-traénoate,
le méthylène [(2-benzoyle-4-méthoxy)-phénoxy]-acétate (tout-E)-9-(4-méthoxy-2,3,6-triméthylphényl)-3,7-di-méthyl-2,4,6,8-nonatétraénoate,
le méthylène cinnamate (tout-E)-9-(4-méthoxy-2,3,6-triméthylphényl)-3,7-diméthyl-2,4,6,8-nonatétraénoate,
le méthylène p-méthoxycinnamate (tout-E)-9-(4-méthoxy-2,3,6-triméthylphényl)-3,7-diméthyl-2,4,6,8-nonatétra-énoate,
l'éthylidène-(éthylcarbonate) (tout-E)-9-(4-méthoxy-2,3,6-triméthylphényl)-3,7-diméthyl-2,4,6,8-nonatétraénoate,
l'éthylidène-benzoate (tout-E)-9-(4-méthoxy-2,3,6-tri-méthylphényl)-3,7-diméthyl-2,4,6,8-nonatétraénoate,
l'éthylidène acétate (tout-E)-9-(4-méthoxy-2,3,6-tri-méthylphényl)-3,7-diméthyl-2,4,6,8-nonatétraénoate.

**8.** Les composés des revendications 1 à 7 en tant que médicaments.

**9.** Utilisation des composés des revendications l à 7 pour la préparation de médicaments pour le traitement des maladies dermatologiques.

**10.** Compositions pharmaceutiques contenant un composé selon les revendications 1 à 7 en tant que substance active et des véhicules pharmaceutiques usuels.

**11.** Procédé de préparation des composés de la revendication 1, caractérisé en ce que l'on fait réagir un acide carboxylique de rétinoîde de formule Ret-OH ou un dérivé réactif d'un tel acide avec un alcool de formule HA ou un dérivé réactif d'un tel alcool, ou bien un halogénure de formule Hal-A, Ret et A ayant les significations indiquées dans la revendication 1 et Hal représentant un halogène, ou bien un composé de formule Ret-O-C($R^4$)$_2$-Hal avec un acide carboxylique de formule $R^{31}$C00H, Ret, Hal et $R^4$ ayant les significations indiquées ci-dessus et $R^{31}$ représentant un groupe alkyle en $C_1$ -$C_6$ , phényle, phényle substitué, styryle ou styryle substitué dans le noyau phényle, en présence d'un agent de condensation, et le cas échéant on soumet les groupes réactifs du produit obtenu à des modifications fonctionnelles.